# EUROPEAN PATENT APPLICATION

(11) **EP 4 137 511 A1**
(43) Date of publication of application: **22.02.2023**
(21) Application number: 20930839.4
(22) Date of filing: 16.04.2020
(51) Int. Cl.: C07K 14/79, A61P 11/00

(54) **NEW COMPOSITION FOR USE TO TREAT AND PREVENT INFECTIONS BY COVID-19 AND OTHER CORONAVIRUSES**

(71) Applicant: Dermopartners, S.L., 46138 Rafelbunyol - Valencia (ES)
(72) Inventor: SERRANO SANMIGUEL, Gabriel, 46138 Rafelbunyol - Valencia (ES)
(74) Representative: Urizar Anasagasti, Jesus Maria
(86) International application number: PCT/ES2020/070245
(87) International publication number: WO 2021/209652

(57) **Abstract**

Disclosed is a novel composition for use to treat and prevent infections by COVID-19 and other coronaviruses, which consists of a food supplement that includes liposome-encapsulated lactoferrin as an essential active ingredient, and other secondary active ingredients, such as a zinc salt and vitamin C, also liposome-encapsulated. Also disclosed is the use of this composition to treat people infected with severe or mild COVID-19 and to prevent infection in non-infected people.

## Description

### Sector of the art

The object of the present invention is applicable in the field of the medical and pharmaceutical industry since it is a composition that is used to prevent and/or treat viral infections, more specifically infections by coronavirus, that have recently generated a pandemic affecting many countries due to the lack of vaccines and/or effective treatments against these viruses.

### State of the art

There are numerous pharmaceutical compositions that incorporate lactoferrin as an active ingredient and several patents are known in this regard, but the vast majority of them refer to dermatological treatments, diabetes treatments, such as patents EP2323625, EP2692355, EP3603621, EP3210618, US2016206707, *et al.*

But there is no known reference to the use of pharmaceutical compositions that incorporate liposome-encapsulated lactoferrin to treat and prevent infections by COVID-19 or other coronaviruses.

### Description of the invention

Based on the prior art, the objective of the present invention is to provide a pharmaceutical composition that can combat and/or prevent infection by coronavirus. The composition of the present invention was obtained by encapsulating its components in liposomes and was tested in a clinical study.

The inventor of this patent is aware of the many effects that lactoferrin has on the human body as the founding president of Sesderma laboratories, which has been marketing for more than 14 years the drinkable product called Lactyferrin^{™} Forte that contains liposomal lactoferrin, and the possible efficacy of this product against COVID-19 was considered for which a clinical study was carried out.

A prospective observational study was conducted in 75 patients with typical COVID-19 symptoms who tested positive for the rapid IgM/IgG test. The patients were isolated and treated at home using remote systems, checked in on twice a day for 10 days and followed up to 1 month.

A bovine liposomal lactoferrin (LLF) syrup feed supplement (32 mg lactoferrin/10 mL plus 12 mg vitamin C) was administered orally in 4 to 6 doses per day for 10 days. Furthermore, a zinc solution was administered at the dose of 10 mg / 10 ml two or three times a day. A control group of 12 patients taking LLF alone was included. All family members in contact with patients (256 people) were also treated with half of this dose. The treatment allowed a complete and rapid recovery in all patients (100%) within the first 4-5 days. The same treatment at lower doses prevented the disease in healthy people directly related to the affected patients. Weakness (95%) followed by a dysfunction in the perception of smells and tastes (88%), cough (83%), muscular pain (67%) were the most frequent complaints.

In December 2019, coronavirus disease 2019 (COVID-19) emerged in the city of Wuhan and spread rapidly throughout China and to five continents in a few months, creating a pandemic. Since then, there has been a desperate search for a safe and effective treatment.

Clinically, the disease starts with a fever, headache, dry cough, fatigue, myalgia, respiratory distress, abdominal pain, diarrhoea, nausea and vomiting. The onset of the disease can lead to progressive respiratory failure due to alveolar damage and even death. Laboratory prognostic factors for adverse clinical outcomes include lymphopenia (35-75% of cases), increased CRP values (75-93% of cases), LDH (27-92% of cases), ESR (up to 85% of cases) and D-dimer (36-43% of cases), as well as low concentrations of serum albumin (50-98% of cases) and haemoglobin (41-50%). Instead, many laboratory abnormalities were predictive of adverse outcomes, including an increased white blood cell count, increased neutrophil count, decreased lymphocyte count, decreased albumin, increased LDH, ALT, AST, bilirubin, creatinine, cardiac troponins, D-dimer, prothrombin time, procalcitonin and CRP values.

Antivirals and hydroxychloroquine were initially suggested against COVID-19-associated pneumonia in multicentre clinical trials conducted in China.

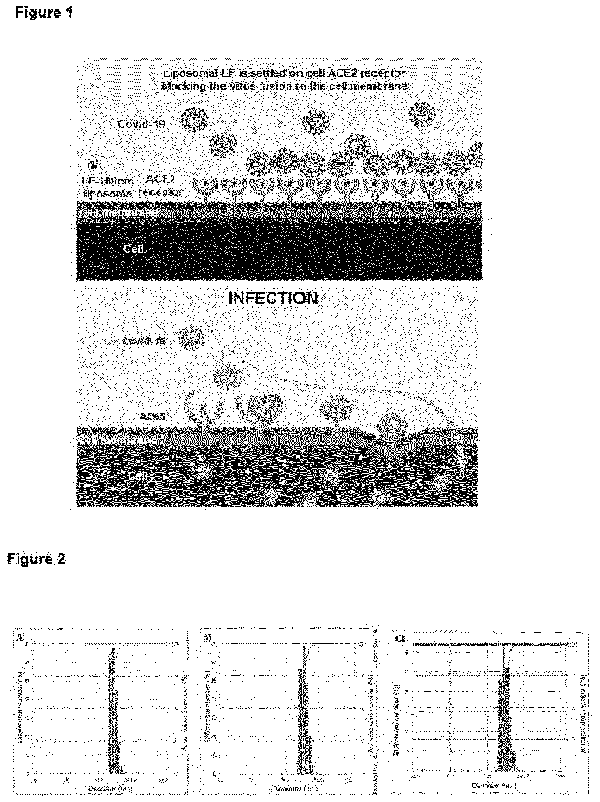

The angiotensin converting enzyme 2 (ACE2) receptor is the main receptor for COVID-19 (Figure 1) and plays a vital role in virus entry into the cell to induce pulmonary infection. These receptors are highly expressed in the nose, mouth, epithelial respiratory tract, the alveolar epithelial cells of the lungs, the enterocytes of the small intestine and the brush border of the proximal tubular cells of the kidney. The ACE2 receptor locations are related to tissue tropism and the pathogenesis of the viral infection. The disease can cause an infection of the upper respiratory tract (sinuses, nose and throat) but most often an infection of the lower respiratory tract (trachea and lungs). COVID-19 infection spreads in the same way as other coronaviruses, primarily through person-to-person contact. Infections range from mild to severe.

In the year 2011, Lang *et al.* (2011) stated that lactoferrin could potentially be useful for the treatment of SARS disease. However, until now, there have been no reports of the clinical use of lactoferrin in patients affected by SARS-CoV or COVID-19 infection.

COVID 19 is an enveloped RNA virus with a genome approximately 30,000 nucleotides in length and encodes a complex of non-structural replicase and structural proteins including, as spike (S), envelope (E) and S2, membrane (M) and nucleocapsid (N) proteins. The spike protein is made up of two units: S1, which mediates binding of the virus to receptors on target cells, and S2, which triggers the fusion of the virus and the host cell membrane. Angiotensin converting enzyme 2 (ACE2), a metallopeptidase, is a functional virus receptor and is responsible for binding to the S protein (Figure 1) and mediating virus entry into target cells. A segment with amino acids 318-510 of the S1 protein is the receptor binding domain for the ACE2 receptor. ACE2 is highly expressed in human lung alveolar epithelial cells, the enterocytes of the small intestine and the brush border of the proximal tubular cells of the kidney. These locations of ACE2 expression are consistent with tissue tropism and the pathogenesis of SARS-Co infection. Some patients with COVID 19 infection develop an intense host immune response against the virus. The innate immune response plays an important role in decreasing viral infection. Many genes may be involved in the innate immune response, such as lactoferrin-coding, S100A9 and lipocalin 2, which participate in the elimination of SARS-CoV.

Lactoferrin gene expression was found to be upregulated and lactoferrin was highly elevated (150-fold) in SARS patients compared to healthy volunteers. In our experience, CSGF was also upregulated, but in contrast, GMCSF was downregulated after healthy volunteers ingested liposomal lactoferrin. It is thought that lactoferrin in this infection may function to stimulate NK cell activity and neutrophil aggregation and adhesion.

Lactoferrin is a multifunctional glycoprotein present in various body fluids including milk, saliva, tears, semen, vaginal fluids, nasal and bronchial secretions, gastrointestinal fluids and urinary mucosal secretions and is also present in the neutrophil granules of leukocytes.

Lactoferrin has strong antiviral activity against RNA and DNA viruses, including human immunodeficiency virus, Zika virus, Chikungunya, hepatitis C, Sindbis virus, cytomegalovirus (herpes simplex virus, human papillomavirus and rotavirus). These viruses use common molecules such as heparan sulphate proteoglycans (HSPG) in the cell membrane to facilitate cell invasion. These molecules provide the first attachment sites on the cell surface and help the virus make primary contact with host cells.

Lactoferrin may be able to prevent internalization of some viruses after binding to HSPG, which is present in most cells. This property of lactoferrin confers protection to the host against viral infections. Lactoferrin has an important protective role in host immune defence against the invasion of COVID 19.

75 patients affected by COVID-19 with typical symptoms of the disease and positive for COVID-19 were selected for inclusion in the study. The patients were isolated and treated at home using remote systems, checked on daily for 10 days, and blood diagnosis of up to 1 month was confirmed by the rapid IgS / IgG SARS-CoV-2 antibody test performed on whole blood (IgM SARS-CoV-2 / Rapid IgG antibody test (Liming Bio, Jiangsu, PR China). LLF (drinkable Lactyferrin^{™} Forte, Sesderma laboratories, Valencia, Spain), as well as a liposomal zinc (LZ) syrup (zinc defence syrup, Sesderma laboratories, Valencia, Spain) were administered orally in 4 to 6 doses per day. The LZ solution was administered at the dose of 10 mg/10 ml two or three times a day. A control group of 12 patients also received LLF alone. The total daily dose of LLF varied between 256-384 mg/day. All family members in contact with the patients (256 people) were also treated with half of this dose. The patients who presented headache, dry cough and nasal congestion were also treated with LL nasal drops and mouth spray (lactoferrin nasal drops and lactoferrin mouth spray) applied 4 times a day. Aerosolized LL (SES Nanomist, Sesderma Laboratories, Valencia, Spain) was also administered to all patients with shortness of breath. All participants were evaluated by the medical team through remote systems. The patients were monitored daily (at least 2 times a day) for 10 days and then 30 days later. The symptoms evaluated in each patient were scored on a scale of 0 to 3, with 0 being the absence of, 1 mild symptom, 2 moderate symptoms and 3 severe symptoms. In the case of the taste and smell symptom, these were evaluated on a scale of 0 to 5, where 0 represents the absence of taste / smell (ageusia / anosmia) and five unaffected taste / smell.

For the group with the combined treatment (LLF + LZ), the mean age of the patients was 42 years and 45% were women. Also included for reference is a special group of four patients who underwent mechanical ventilation and were treated in hospital. All were released after 10 days. None died. The most common symptoms were weakness/tiredness (94.44%), loss of smell (83.33%) and taste (88.89%), muscular pain (66.67%), dry cough (61.11%), headache (55.56%), diarrhoea (44.4%), nasal secretion (33.33%), shortness of breath (27.78%), nasal congestion (22.22%) and odynophagia (22.22). Symptoms include fever (38%), cramps (30%), insomnia (50%), nightly agitation (30%), nausea and severe stomach pain, flatulence, sore throat (28%) and one patient complained of severe and abrupt hair loss (1.3%). No analytical data (X-ray or computed tomography) were collected. Patients treated in hospital were not included due to lack of a complete data set.

**DAY 0:** Data collected (symptoms) in the first session of the remote system with the participants. They have not yet started treatment (Table 1).

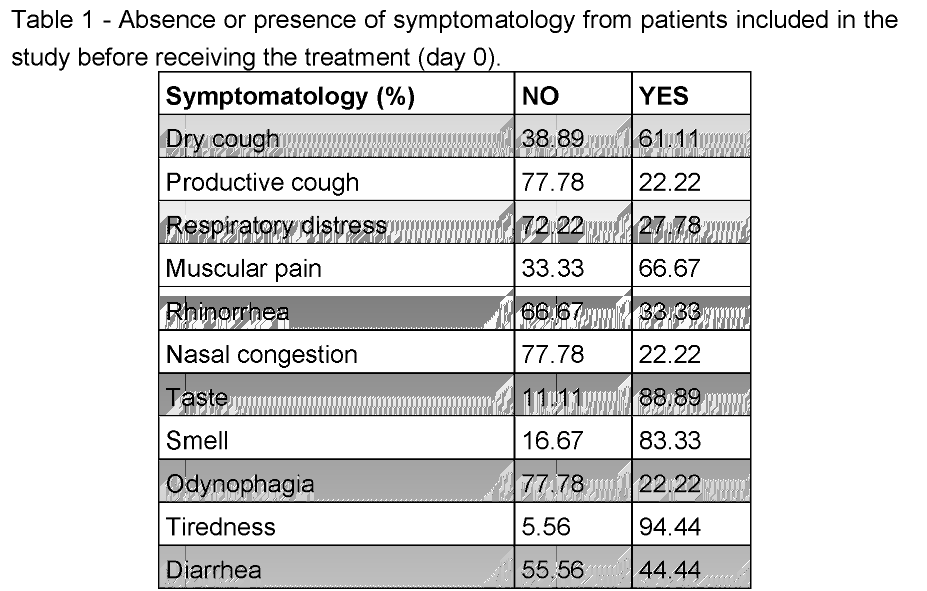

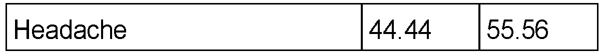

The percentages of each of the symptoms observed according to the severity scale are shown below (Table 2). The parameters evaluated were already mentioned.

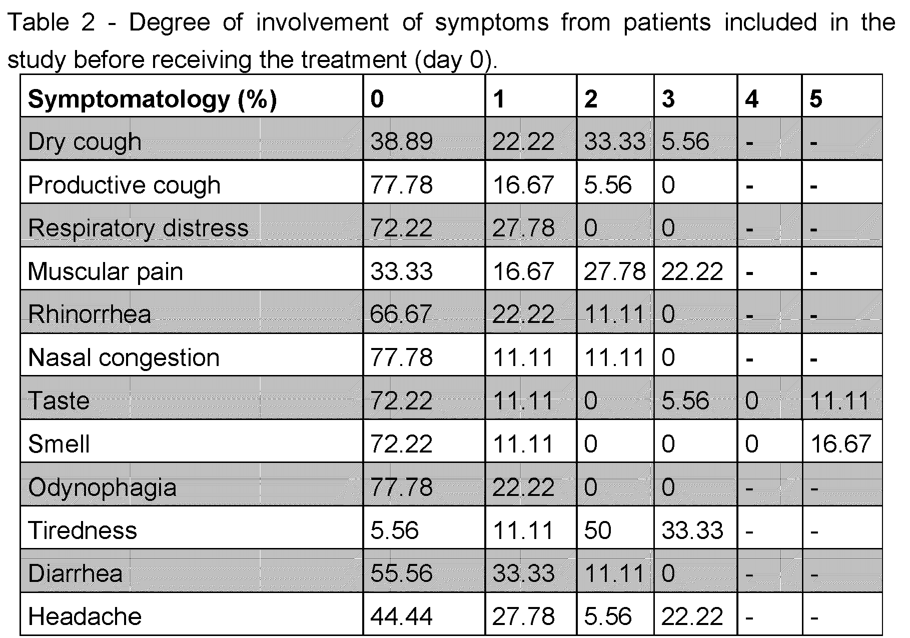

**48 HOURS:** Data collected 48 hours after the first contact with the participants. Patients had already started treatment and improvement was beginning to be seen. (Table 3).

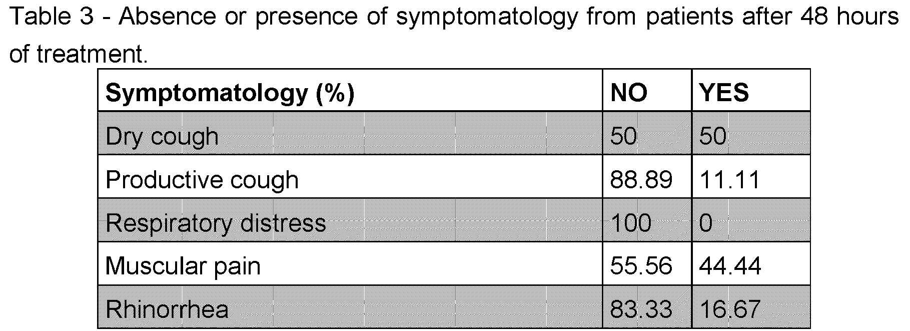

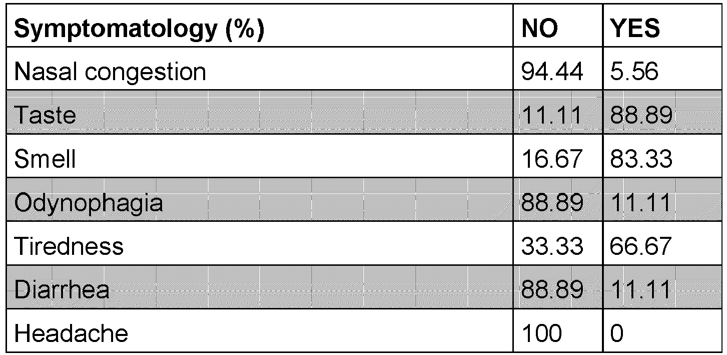

**After the first 48 hours** of taking the treatment, headache disappears in 100% of patients. Dry cough decreases from 61.11 % to 50%. These patients found significant relief from these symptoms and it was related to the application of nasal drops and spray. Muscle pain was reduced from 66.67% to 44.44%. Tiredness/weakness decreased from 94.44% at the start of the study to 66.67% after 48 hours. In all patients with moderate to severe respiratory distress, lactoferrin nebulization was performed with a Nanomist nebulizer (SES Nanomist, Sesderma laboratories, Valencia, Spain). In the case of taste and smell, there was no significant improvement in these symptoms after 48 hours (Table 3). The patient's recovery of smell and taste was slower compared to the rest of the symptoms. In Table 4 (% severity scale for symptoms), it was observed that 72.22% of the patients who showed total absence of taste and smell (ageusia / anosmia) on day 0) (Table 1), presented a reduction at 48 h (44.44% and 38.89% for taste and smell).

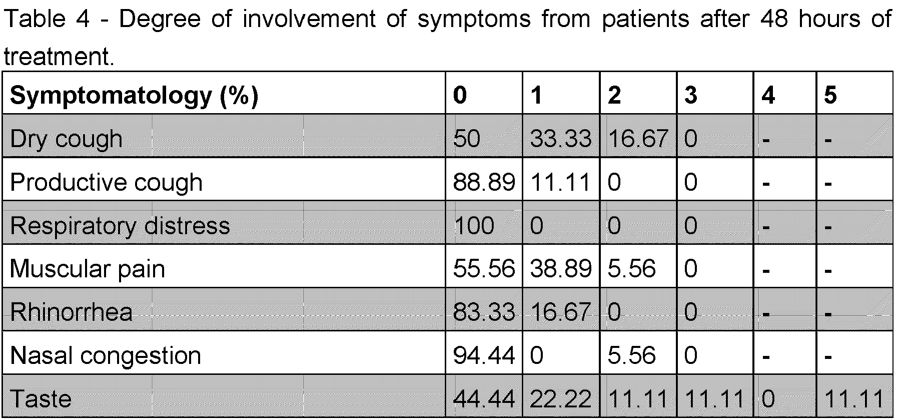

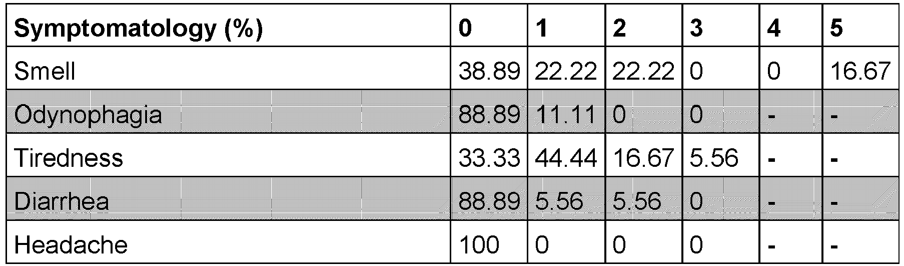

**Day five.** All participants continued to improve and continued the scheduled 10-day treatment. On the fifth day, dry cough decreased from 61.11% to 38.89%. Muscle pain was reduced from 66.67% to 22.22%. Tiredness/weakness decreased from 94.44% to 27.78%. Headache remained absent in 100% of patients after 48 hours and on the fifth day. Loss of taste and smell (Table 5) did not show a significant effect, since it was observed with the rest of the symptoms. Patients' recovery of smell and taste was slower.

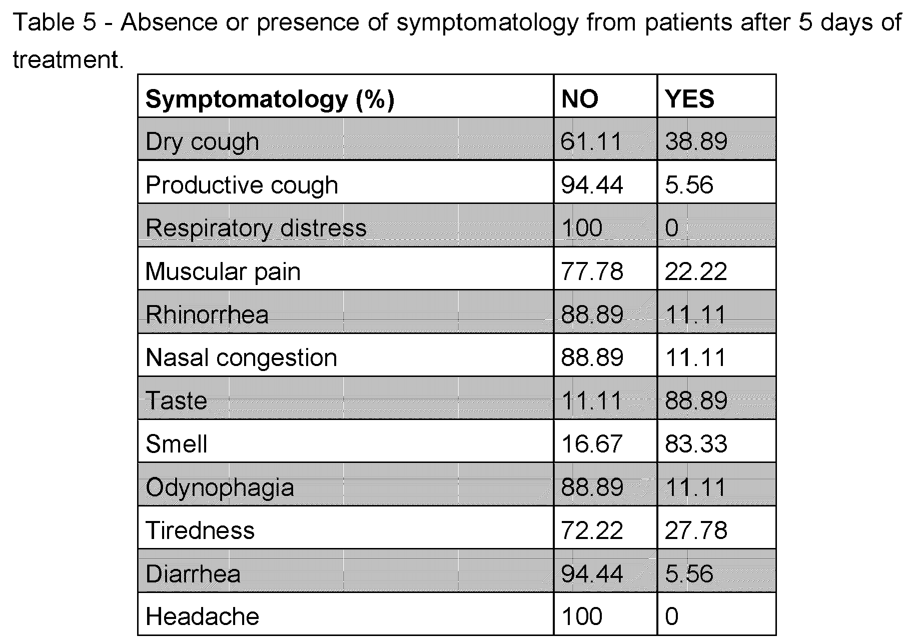

However, Table 6 (% degree of severity of symptoms) shows a progressive improvement in symptoms. Of the approximately 72.22% of the patients who showed total absence of taste and smell (ageusia/anosmia) on day 0, the percentage dropped to 38.85 on day 5.

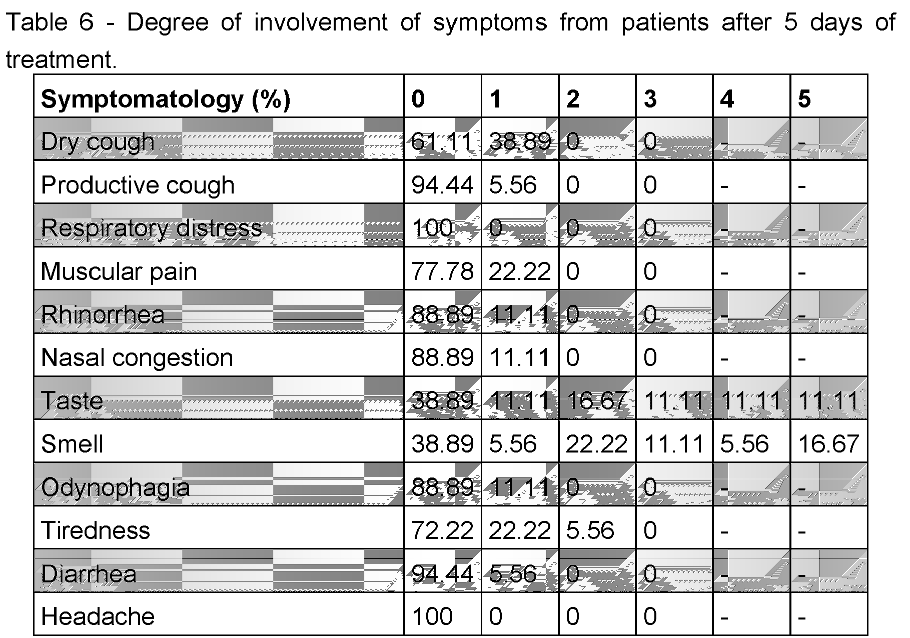

The evolution of the patients' main COVID-19 symptoms on day 0, 48 h and 120 after 48 hours (day 5) is described below.

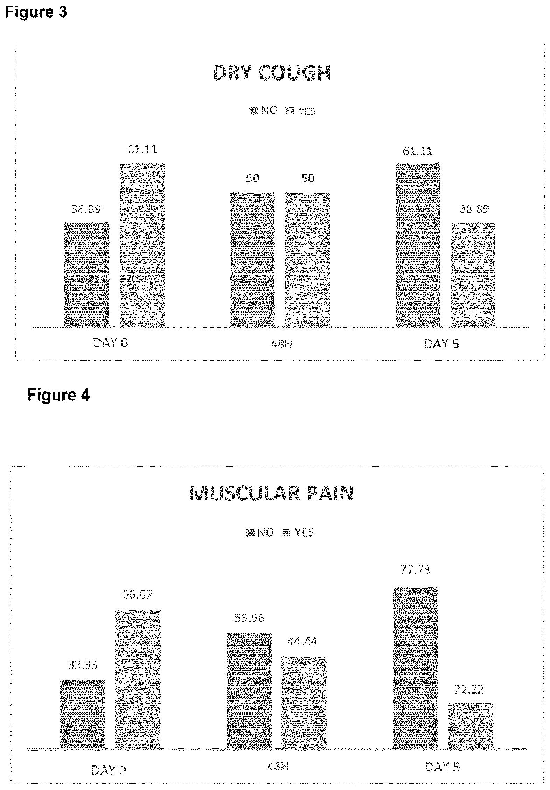

**DRY COUGH** (Figure 3): At the beginning of the study (day 0), 61.11 % of the patients had a dry cough. After 48 and 120 hours of treatment, this percentage was reduced to 50% and 38.89%, respectively. The treatment markedly reduced dry cough.

**SHORTNESS OF BREATH** (Table 3): 100% of patients with shortness of breath were completely improved at 48 hours.

**MUSCULAR PAIN** (Figure 4): at the beginning of the study (day 0), 66.67% of the patients had muscular pain. After 48 and 120 hours, this percentage was reduced to 44.44% and 22.22%. The treatment significantly reduced severe muscular pain associated with COVID-19.

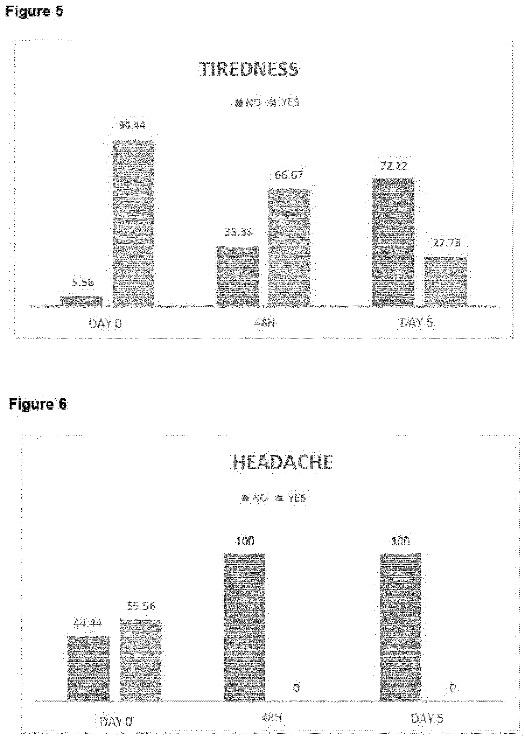

**TIREDNESS** (Figure 5): 94.44% of the patients were tired on day 0. After 48 and 120 hours of treatment, this percentage was reduced to 66.67 and 27.78%, respectively.

**HEADACHE** (Figure 6): On day 0, 55.56% of the patients presented severe headache. After 48 and 120 hours of treatment, the headache completely disappeared in 100% of the patients. At 120 hours the patients remained without headache.

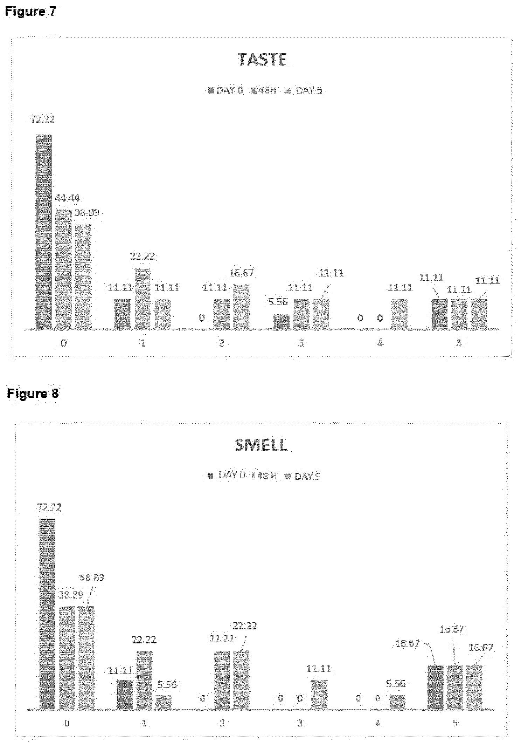

**TASTE** (Figure 7): On day 0, 72.22% of the patients had ageusia (total absence of taste), while 11.11% of the patients had no taste involvement. The rest of the patients had hypogeusia (partially decreased sense of taste). After 48 and 120 hours of treatment, the percentage of patients with ageusia decreased to 44.44% and continues to decrease to 38.89%, which implies a partial and progressive recovery of taste. At 10 days, all patients fully recovered smell and taste. Most of the patients complained of not recognising the taste of food (sweets, seafood, spicy chili pepper).

**SMELL** (Figure 8): On day 0, 72.22% of the patients had anosmia (total absence of smell), while 16.67% of the patients had no olfactory compromise. The rest of the patients had hyposmia (partially decreased sense of smell). After 48 hours, the percentage of patients with anosmia decreased to 38.89 and remained the same at 120 hours. These alterations completely improved in 95% of cases by day 10.

The results obtained in the control group that took LLL were very similar. After analysing these results, it can be concluded that oral treatment with LLF and LLF + LZ allows rapid recovery in all patients (100%) within the first 4-5 days. The same treatment at lower doses seems to prevent the disease in healthy people directly related to the affected patients. It is important to note that the combination of oral treatment with topical treatment of the nose and mouth provided significant relief of headache and cough. Some of these patients had sinus congestion. Lactoferrin and zinc dietary supplements enhanced and supported the immune system response through their antioxidant, antibacterial and antiviral properties.

The most frequent symptom in our patients was a very intense feeling of tiredness or weakness (95%) followed by an altered perception of smell and taste (88%), cough (83%), muscular pain (67%), headache (56%) and diarrhoea (44%). Most symptoms improved significantly during the first five days by a percentage of improvement for headache (100%), muscular pain (78%), taste (72%), smell (17%), weakness (72%) and dry cough (61%). At day, all patients were asymptomatic and complete recovery was observed in all persons (100%). Olfactory and taste dysfunction was the most predominant symptom in the present cases and occurred very early, reflecting the massive entry of viral particles into the oral and nasopharyngeal mucosa.

These alterations were described in severe acute respiratory syndrome (SARS). Patients could not recognise the taste of food, not even sweet or spicy ones, or smell fragrances (anosmia) or household cleaners (alcohol, kitchen detergents or chlorine bleach). The angiotensin converting enzyme 2 (ACE2) receptor, which are the main receptors for COVID-19, are highly expressed in the nose, mouth, particularly at the base of the tongue and throat sites for which the virus has a high tissue tropism.

Topical care of the nose, mouth and throat with nasal drops, mouthwash, throat sprays and even liposome-based toothpastes could help reduce viral load. These alterations can be considered a transient olfactory and taste dysfunction and require further follow-up and research. Patients receiving chemotherapy can improve taste and smell dysfunction by taking lactoferrin, improving oral immunity of the cancer patient.

Treatment of using LL nasal drops was found to be very helpful in relieving not only respiratory symptoms but also cough, headache and smell and taste dysfunction. 28% of the patients presented shortness of breath. In these cases, use of the lactoferrin liposomal aerosol inhalation device was recommended 4 times a day with great success. Inhalation was well tolerated and no side effects were reported. Inhalations via the LLF nebulizer were also recommended for two of the four patients admitted to hospitals. Lactoferrin is a natural protein present in bronchial secretions and the entire content of the liposome is biocompatible and biodegradable.

Aerosolized liposomal therapy has been used for years with good results and a broad safety profile. It has been used with ribavirin in respiratory syncytial virus infection and in rhinovirus infections. Lactoferrin is a hydrophilic chemical in which, due to encapsulation in phosphatidylcholine, liposomes behave as amphiphiles. Lactoferrin reaches sufficiently high concentrations within the lungs and provides a delayed release of lactoferrin in the target organ, the respiratory tract.

Lactoferrin nanoliposome is 100 nm in size, but once it is nebulized through the airways, it has a reduced size of 50 nm because in the hole of the device there is a filter that reduces the size of the nanosome. The liposome alone can interact with the natural surfactant (phospholipids and proteins) of the target tissue (lungs), and the phosphatidylcholine can also exert a strong anti-inflammatory effect.

Pulmonary infections from COVID-19 can alter total pulmonary surfactant and change its composition, thus decreasing the availability of phospholipids that decrease lung function as occurs in chronic obstructive pulmonary disease. Without a surfactant, the alveoli tend to collapse during normal tidal breathing, resulting in decreased lung compliance.

Lactoferrin is a globular protein belonging to the transferrin family and shows a high affinity for iron ions. Lactoferrin is a defence protein found in human milk, where it is present in relatively high concentrations (1 g/L), especially in the colostrum stage (up to 7 g/L). It is also present in other body fluids (tears, semen, vaginal fluid, saliva, bronchial secretions, etc.). It is synthesized by blood neutrophils and various types of cells, including some acinar cells. Endogenous lactoferrin levels increase during infection and inflammation.

Lactoferrin has antibacterial and antiviral properties, modulates the immune system and protects against intestinal inflammation. Lactoferrin can influence leukocytes by increasing the activity of NK cells, neutrophils and macrophages. It increases the production of cytokines and nitric oxide and limits the growth of pathogens. Promotes the maturation of immune cells, T and B lymphocytes. It may reduce allergy symptoms by blocking the release of histamine from intestinal mast cells. It may likely be useful in preventing and treating the effects of obesity and may promote the reduction of visceral fat. It can help improve the condition of the skin in ailments such as acne, skin inflammation and psoriasis.

In the present study, liposomal lactoferrin (LLF), registered as a food supplement, Lactyferrin^{™}, is used, and this product is found to be safe and effective to treat and prevent COVID-19 as all 75 patients positive for COVID-19 infection were successfully treated. Most patients responded within the first 3 to 4 days of treatment with Lactyferrin^{™}, although treatment was recommended for 10 days.

Individuals in contact with symptomatic patients were also treated with half the curative dose, which resulted in the prevention of the disease. The combination of liposomal lactoferrin and liposomal zinc is very effective and safe for the treatment of this life-threatening disease.

The bovine liposomal nutritional food supplement based on lactoferrin, Lactyferrin^{™} Forte (Sesderma Laboratories, Valencia, Spain) is registered in the European Union (Ireland), as well as by the United States Food and Drug Administration, as a food supplement. The European Food Safety Authority recognises bovine lactoferrin as a dietary supplement with medicinal properties, and it is classified as a novel and safe food ("generally recognised as safe"), with no contraindications.

Total adult doses of unencapsulated lactoferrin range from 1.4 to 3.4 g, and this supplement is frequently used to strengthen the immune system. Lactyferrin^{™} contains lactoferrin (32 mg / 10 ml and vitamin C 12 mg / 10 ml). Both substances are hydrophilic and have very limited gastric absorption. The liposome, with its closed phospholipid bilayer vesicular system, could encapsulate both hydrophilic drugs (lactoferrin, vitamin C, zinc) and hydrophobic drugs (vitamin A).

The liposomes manufactured to obtain the composition of the present patent are based on phosphatidylcholine (PC), which is a biocompatible and biodegradable chemical. Lactoferrin in its free form is degraded in the stomach by the action of hydrochloric acid and hydrolytic enzymes (proteases, pepsin); therefore, the bioavailability of the free form is very limited.

Therefore, lactoferrin and vitamin C are encapsulated in a 100 nm nanoliposome or lipid bubble, made from soy phosphatidylcholine (PC) in Lactyferrin^{™}. The nanoliposome protects lactoferrin from destruction by digestive secretions and allows the intact protein to travel through the duodenum and into the general circulation, from where its bioavailability is very high.

Lactoferrin encapsulated in this way is protected from pepsin and protease hydrolysis. It is also important to note that free lactoferrin is rapidly cleared from the circulation, which limits its therapeutic potential. Therefore, it is necessary to encapsulate it in liposomes to improve plasma stability.

The PC used to manufacture liposomes is a ubiquitous and natural phospholipid molecule, which is the main lipid in cell membranes and blood proteins. Furthermore, PC serves as the main physiological source of choline, an essential nutrient and neurotransmitter precursor, acetylcholine. PC is also needed to produce surfactants, which are critical for pulmonary function and gastrointestinal health. The terms "phosphatidylcholine" and "lecithin" are sometimes used interchangeably; however, lecithin is a mixture of various lipids and phospholipids. PC is necessary for the composition and repair of cell membranes and is vital for normal liver function. Research indicates that PC has a beneficial role in the prevention and treatment of various forms of liver disease and toxicity. PC protects liver cells from viral damage, reduces fibrosis and prevents cell death by drugs, alcohol and other chemical toxins.

The protective effects of lactoferrin range from anticancer, anti-inflammatory and immunomodulatory properties, to antimicrobial, antifungal and antiviral activities against many microorganisms. This wide range of activities is possible thanks to mechanisms of action that involve not only the ability of lactoferrin to bind iron, but also the interactions of lactoferrin with molecular and cellular components of hosts and pathogens.

The antibacterial activity of lactoferrin is related to environmental iron deprivation, which is essential for bacterial growth, while its antiviral activity is associated with its role as a competitor for cell membrane receptors commonly used by viruses to enter cells.

Specifically as stated before, lactoferrin is an ACE2 blocker, and prevents the binding of the S protein of the virus to the host cell, blocking the fusion of the virus with the cell membrane. Liposomal lactoferrin can also suppress viral replication after cell entry, as in the case of HIV. Furthermore, some patients infected with HIV-1 show decreased levels of plasma lactoferrin and in others, the lack of lactoferrin (and secretory IgA) found in the oral cavities of people with HIV strongly correlated with frequent infections in those areas.

Nanoliposomes also have beneficial effects related to their size and composition (PC); they are smaller (100 nm) than the virus (150 nm), can compete to reach target cells, where they settle before the virus does.

It has also been shown that specific doses of liposomal lactoferrin can prevent COVID-19. Lower doses do not prevent infection (unpublished data).

Furthermore, the use of lactoferrin has contributed to the successful treatment of at least four intubated hospitalized, high-risk patients (high ferritin, IL-2, DD2 levels) and with practically absent vital signs. Lactoferrin significantly reduces the hyperimmune reaction seen in critically ill patients suffering from an aggressive storm of proinflammatory cytokines (IL-2 and 6), normalizing or lowering IL-6, TNF alpha, ferritin and DD2 parameters, and protecting lungs against acute respiratory distress. Lactoferrin has immunomodulatory and anti-inflammatory properties, which are important in the pathophysiology of serious infections. Furthermore, lactoferrin has a crucial immunomodulatory role in maintaining immunological and physiological homeostasis and in limiting tissue damage by modulating the cytokines, chemokines and cell surface receptors involved in signalling cascades. The restorative and homeostatic roles of lactoferrin are notable in the context of the "systemic inflammatory response", which describes the physiological response to severe attacks such as sepsis. The concept of "cytokine storm" reflects the hyperinduction of inflammatory responses resulting from uncontrolled immune activation, and these clearly respond to the oral administration of lactoferrin.

Lactoferrin is useful for the treatment of the most severe cases of COVID-19, due to its ability to modulate exaggerated immune and inflammatory responses to viral infections, as observed in at least four patients. LLF might play a role in iron metabolism alterations observed during inflammation and conditions with increased neutrophil turnover. In this case, many released cytokines and/or inflammatory products such as interleukin 1, endotoxin, TNF-alpha and immune complexes can trigger the release of lactoferrin from neutrophils. Lactoferrin will bind to its membrane receptor on monocytes/macrophages and, trapped in this position, could prevent iron transfer from the macrophage to serum transferrin. Iron released from senescent phagocytosed red blood cells could thus be captured by membrane-bound lactoferrin and transferred back to intracellular ferritin.

Liposomal lactoferrin has been used to treat various medical conditions for the past 14 years. The neuroprotective effects of liposomal lactoferrin were also tested in a model of *Caenorhabditis elegans* by evaluating both phenotypic and transcriptomic responses. The lactoferrin-based product protected against acute oxidative stress and extended the shelf life of C. *elegans* in a dose-dependent manner. Furthermore, the paralysis of the transgenic strain of C. *elegans,* CL4176, caused by Aβ1-42 aggregates, was clearly improved by lactoferrin treatment. Analysis of the transcriptome of treated nematodes indicated that it led to stimulation of the immune system, along with the improvement of the processes involved in the response to oxidative stress. The lactoferrin-based product also enhanced processes involved in protein homeostasis, cell adhesion and neurogenesis in the nematode. Therefore, it was concluded that lactoferrin provides protection against aging and neurodegeneration, modulating the processes involved in the response to oxidative stress, protein homeostasis, synaptic function and xenobiotic metabolism. The lactoferrin-based liposomal product was also able to stimulate the immune system, as well as improve reproductive status and energy metabolism. Together, all these findings suggest that oral supplementation with liposomal lactoferrin could benefit the immune system and improve antioxidant capacity.

Many patients admitted to local hospitals in Spain with COVID-19 are over the age of 70 and have very low levels of zinc, which can contribute to the severity of the infection. Zinc is also hydrophilic and poorly absorbed through the gastrointestinal tract. Curiously, the administration of nano-encapsulated zinc could support the recovery of patients with COVID-19 infection.

Zinc has also exhibited a powerful antiviral effect. In experiments with poliovirus, zinc inhibited viral infection when incubated with cells after viral fusion, and the level of inhibition was correlated with the degree of zinc saturation. Zinc supplements have previously been proposed for administration to patients with COVID-19. Zinc may also influence metalloproteases involved in the fusion process of the coronavirus, by decreasing both cell entry and cell-cell fusion.

Lactoferrin can be used together or in combination with zinc and both supplements are non-toxic and can also be used as adjunctive treatments, along with conventional antiviral drugs or hydroxychloroquine, as shown for the treatment of hepatitis C virus (HCV), where lactoferrin decreased the HCV RNA titer by contributing to the effectiveness of combined interferon and ribavirin therapy.

Lactoferrin has great potential for use as a complementary treatment for patients with viral diseases. Based on experience in the clinical study conducted with COVID-19 home-isolated patients and careful contact tracing, it can be concluded that liposomal lactoferrin can prevent and cure infection in a dose-dependent manner. This treatment is also indicated in patients with severe disease, as observed in four patients who were seriously ill. Doses recommended to treat and prevent COVID-19 are provided in the preferred embodiment. This mentioned treatment is completely free from side effects. Some institutions are promoting empiric treatment with hydroxychloroquine plus azithromycin. However, clinical studies are not complete and hydroxychloroquine is not without side effects and can induce a wide range of adverse effects. Cardiovascular, dermatological, gastrointestinal, haematological, hepatic, hypersensitivity, metabolic, musculoskeletal, nervous system, ocular, psychiatric and respiratory side effects have been reported. Particularly, cardiac rhythm disturbances have been observed in a patient with systemic lupus erythematosus who developed syncopal episodes as a result of significant prolongation of the QT interval. This was corrected after discontinuation of the drug. The administration of azithromycin is a paradox. Antibiotics have no effect on the virus, and the drug is given due to its immunomodulatory effect, but unfortunately azithromycin shares with hydroxychloroquine the potential to induce QT prolongation by causing abnormal changes in the electrical activity of the heart that can lead to a life-threatening irregular cardiac rhythm.

Antibiotics alter the gut microbiota that has already been destroyed by COVID-19 and administration of probiotics is recommended. Elderly patients taking antibiotics may develop intestinal dysbiosis with changes in the intestinal microbiota, which makes these patients prone to heart failure.

In the clinical study carried out, 94.44% of the patients developed diarrhoea which LLF counteracts before the fifth day. LLF can exert an anti-inflammatory effect both at the gastrointestinal level that balances the local microbiota, and reduces intestinal damage induced by the virus. Lactoferrin increases good micro flora, such as bifidus, and decreases bad bacteria, such as *E. coli.,* streptococcus, clostridium, *et al.*

Lactoferrin acts as an anti-inflammatory agent promoting "good" cytokines such as interleukin (IL)-4 and IL-10 and reducing pro-inflammatory cytokines such as tumour necrosis factor alpha, IL-6 and IL-1 beta, and the negative regulation of nuclear factor-kappa. The best thing about lactoferrin treatment is that it is simple, safe and quite effective in COVID-19 infections, both in young adults, elderly people and in children, women and men, regardless of the underlying pathology.

Also noteworthy is the mediation by lactoferrin of inflammation induced by COVID-19 infection leading to activation of macrophages and dendritic cells. Neutrophils degranulate at the site of injury and release massive amounts of LF. The LF in turn can increase low responses or modulate ultra-aggressive cytokine activity. Both of these effects serve to control inflammation and assist in tissue repair after trauma.

Lactoferrin needs to be encapsulated in a PC liposome to protect it from pepsin hydrolysis and the acidic pH of the stomach which allows the glycoprotein to pass completely through the intestines and be absorbed.

To complement the description that is being made and for the purpose of helping to facilitate the understanding of the features of the invention, accompanying this specification is a set of drawings in which, the following has been depicted with an illustrative and non-limiting character:
**Figure 1** - Two images showing how the composition prevents coronavirus from entering the cell and when there is infection.
**Figure 2** - Particle size distribution by number of particles (%) obtained by DLS studies for **A)** lactoferrin liposomes, **b)** sodium ascorbate liposomes, and c) zinc sulphate liposomes.
**Figure 3** - Absence or presence of dry cough in patients before treatment (day 0) and after treatment (48 hours and 5 days).
**Figure 4** - Absence or presence of muscular pain in patients before treatment (day 0) and after treatment (48 hours and 5 days).
**Figure 5** - Absence or presence of tiredness in patients before treatment (day 0) and after treatment (48 hours and 5 days).
**Figure 6** - Absence or presence of headache in patients before treatment (day 0) and after treatment (48 hours and 5 days).
**Figure 7** - Absence or presence of taste in patients before treatment (day 0) and after treatment (48 hours and 5 days).
**Figure 8** - Absence or presence of smell in patients before treatment (day 0) and after treatment (48 hours and 5 days).

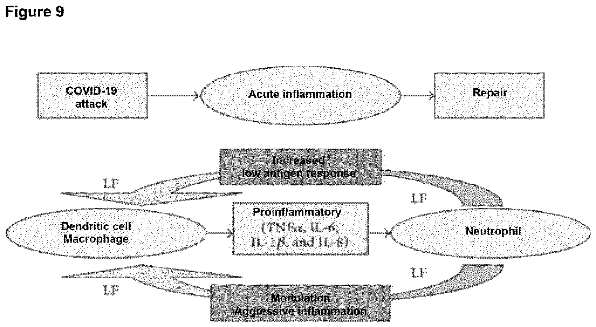

**Figure 9** - Diagram depicting mediation by lactoferrin of inflammation induced by COVID-19 infection leading to the activation of macrophages and dendritic cells.

### Description of an embodiment

A method of carrying out the invention is set forth below, on the one hand regarding the composition object of the present invention and on the other hand the use thereof.

Food grade soy lecithin was purchased from Lipoid (Ludwigshafen, Germany); tween 20 was purchased from Comercial Química Massó (Barcelona, Spain); 96° ethanol was purchased from Panreac (Barcelona, Spain); lactoferrin was purchased from Ferrer Health (Barcelona, Spain); sodium ascorbate was purchased from Fagron Ibérica (Barcelona, Spain); and zinc sulphate heptahydrate was purchased from Nutrifoods (Barcelona, Spain).

The different liposomal solutions were prepared according to the procedure developed by the Sesderma laboratories (Valencia, Spain).(Serrano et al., 2015) Specifically, phospholipids (phosphatidylcholine) were dissolved in 96° ethanol at room temperature, and subsequently tween 20 was added to the ethanolic solution. In parallel, the different active ingredients were dissolved in bidistilled water at the concentration of 5.2, 150 and 14 mg/ml for lactoferrin, sodium ascorbate and zinc sulphate, respectively. Subsequently, the lipid mixture and the aqueous solution containing the corresponding active ingredient (lactoferrin, sodium ascorbate or zinc sulphate) were mixed together and vigorously stirred for 5 minutes. Lastly, the resulting liposomal suspensions were filtered through a surfactant-free cellulose acetate syringe filter with a 0.2 µm pore size.

The final concentration of the active ingredients in the different liposomal solutions is detailed in the following table:
Concentrations of active ingredient in the different liposomal solutions prepared.

| Active ingredient | Concentration (mg/ml) |
|---|---|
| lactoferrin | 4 |
| Sodium ascorbate | 100 |
| Zinc sulphate | 10 |

The characterisation of the liposome in terms of particle size was evaluated by dynamic light scattering (DLS). All measurements were performed with a Delsa Nano C particle analyser (Beckman Coulter, Madrid, Spain), and performed in triplicate on diluted suspensions of the liposomes with filtered deionized water. The polydispersity index (PDI) was used as a measure to define the homogeneity of the size distribution. A sample with a PDI value equal to or less than 0.2 is considered a homogeneous population.

In order to administer the encapsulated active ingredients to the study volunteers, the prepared liposomal solutions were included in two liquid food supplement formulations. The food supplement formulations that consisted of a mixture of components with a nutritional or physiological effect are listed in the following Table:
Composition of food supplements.

| **Food supplement 1** | **Food supplement 2** |
|---|---|
| Citric acid | Citric acid |
| Potassium sorbate | Potassium sorbate |
| Sucralose | Sucralose |
| Flavouring | Flavouring |
| Sodium benzoate | Sodium benzoate |
| Xanthan gum | Xanthan qum |
| Glycerin | Glycerin |
| Sodium ascorbate liposomes | Zinc sulphate liposomes |
| Lactoferrin liposomes | |

The concentration of the active ingredients in the different formulations of food supplements is specified in the following table:
Concentrations of active ingredient in the prepared formulation of the food supplement (SA).

| Active ingredient | Concentration (mg/ml) | |
|---|---|---|
| | SA1 | SA2 |
| lactoferrin | 3.2 | - |
| Sodium ascorbate | 1.2 | - |
| Zinc sulphate | - | 1.1 |

Particle size studies revealed that all of the liposome formulations used in this study have a particle diameter in the range of about 100-130 nm, with a PDI below 0.15. The results are shown in Figure 2 and summarized in the following Table:
Particle diameter measured by DLS and polydispersity index (PDI) of lactoferrin, sodium ascorbate and zinc sulphate liposomes.

| Liposome-encapsulated active ingredient | Particle diameter (nm) | PDI |
|---|---|---|
| lactoferrin | 124 ± 6 | 0.14 ± 0.03 |
| Sodium ascorbate | 128 ± 8 | 0.06 ± 0.02 |
| Zinc sulphate | 130 ± 4 | 0.11 ± 0.03 |

As regards the use of this composition, it should be done at the following doses of liposomal lactoferrin or amount of composition:
Curative doses: 64-96 mg (20-30 ml) every 6 h daily to cure COVID-19 infection (256-384 mg/day). Doses can be increased to 640 to 960 mg/day.
Preventive doses: at doses of 64 mg two or three times a day, it prevents COVID-19 infection (128-192 mg/day).

Pregnancy lactoferrin and infant syrup (alcohol-free glycerosome encapsulation):
- Pregnant women and infants under two years of age.
- Mothers: 20 mg (6.25 ml) three or four times a day (72-80 mg/day).
- Infants: 20 mg (6.25 ml) twice a day.
- Zinc defence syrup: 10-30 mg/day (10-30 ml)

Likewise, regarding the nasal administration of lactoferrin nasal drops (Lactyferrin^{™}): these nasal drops contain nano lactoferrin to quickly relieve the acute sinusitis and alternations in taste and odor experienced by many patients, while at the same time contributing to the management of dry cough. In acute cases, 2 drops applied in each nostril every 2-3 hours for 12 hours is recommended, after that every 4-6 hours. Lactoferrin nasal drops are a very important factor in relieving respiratory symptoms.

Having sufficiently described the nature of the invention, as well as an exemplary preferred embodiment, it is stated for the appropriate purposes that the materials, shape, size and arrangement of the elements described may be modified, provided that this does not imply an alteration of the essential features of the invention that are claimed below.

## Claims

1. A composition for use to treat and prevent infections by COVID-19 and other coronaviruses, **characterised in that** the main active ingredient is liposomal lactoferrin.

2. The composition for use to treat and prevent infections by COVID-19 and other coronaviruses according to claim 1, **characterised in that** the concentration of liposomal lactoferrin in the composition is between 2.9 mg/ml and 3.5 mg/ml and preferably 3.2 mg/ml.

3. The composition for use to treat and prevent infections by COVID-19 and other coronaviruses according to claim 1, **characterised in that** the diameter of the lactoferrin liposome particle is 124 ± 6 nm.

4. The composition for use to treat and prevent infections by COVID-19 and other coronaviruses according to claims 1 and 3, **characterised in that** the phospholipid with which the lactoferrin liposome is made is phosphatidylcholine.

5. The composition for use to treat and prevent infections by COVID-19 and other coronaviruses according to claim 1, **characterised in that** in addition to the main active ingredient, which is lactoferrin, it contains a liposome-encapsulated zinc salt at a concentration between 1.0 mg/ml and 1.2 mg/ml and preferably 1.1 mg/ml.

6. The composition for use to treat and prevent infections by COVID-19 and other coronaviruses according to claim 5, **characterised in that** the zinc salt is preferably zinc sulphate.

7. The composition for use to treat and prevent infections by COVID-19 and other coronaviruses according to claims 1, 5 and 6, **characterised in that** in addition to the main active ingredient, which is lactoferrin, and another secondary active ingredient, such as zinc salt liposomes, it contains vitamin C liposomes at a concentration between 1.1 mg/ml and 1.3 mg/ml and preferably 1.2 mg/ml.

8. A use of the composition of the preceding claims to treat and prevent infections by COVID-19 and other coronaviruses at the following doses of liposomal lactoferrin:
- curative doses: 64-96 mg (20-30 ml of composition) every 6 h daily to cure COVID-19 infection (256-384 mg liposomal lactoferrin/day);
- preventive doses: 64 mg (20 ml of composition) two or three times a day (128-192 mg liposomal lactoferrin/day).

9. A use of the composition of the preceding claims to treat and prevent infections by COVID-19 and other coronaviruses at doses of liposomal lactoferrin that can be increased up to 960 mg/day.

10. A use of the composition of the preceding claims to treat and prevent infections by COVID-19 and other coronaviruses in pregnant women and infants under two years of age at the following doses of liposomal lactoferrin:
- mothers: 20 mg (6.25 ml) three or four times a day (72-80 mg/day);
- infants: 20 mg (6.25 ml) twice a day.
